Europäisches Patentamt

European Patent Office

Office européen des brevets

(11) Numéro de publication: **0 409 698 A1**

# DEMANDE DE BREVET EUROPEEN

(21) Numéro de dépôt: 90402013.8

(51) Int. Cl.5: **A61B 6/00**

(22) Date de dépôt: **12.07.90**

(30) Priorité: **20.07.89 FR 8909781**

(43) Date de publication de la demande:
**23.01.91 Bulletin 91/04**

(84) Etats contractants désignés:
**DE ES GB IT NL**

(71) Demandeur: **GENERAL ELECTRIC CGR S.A.**
**100, rue Camille-Desmoulins**
**F-92130 Issy les Moulineaux(FR)**

(72) Inventeur: **Feldman, Andréi**
**Cabinet Ballot-Schmit, 7 rue le Sueur**
**F-75116 Paris(FR)**
Inventeur: **Giudici, Philippe**
**Cabinet Ballot-Schmit, 7 rue le Sueur**
**F-75116 Paris(FR)**

(74) Mandataire: **Ballot, Paul Denis Jacques et al**
**Cabinet Ballot-Schmit 7, rue le Sueur**
**F-75116 Paris(FR)**

(54) **Procédé de correction de la mesure de la densité osseuse dans un scannner.**

(57) L'invention concerne les scanners à rayons X et, plus particulièrement, un procédé de mesure de la densité osseuse d'un patient qui tient compte de la corpulence de ce dernier.

Le procédé de l'invention consiste à effectuer un étalonnage de la mesure de la densité osseuse à l'aide d'un fantôme 37 simulant le corps du patient et la vertèbre (insert 39) et d'un fantôme d'étalonnage comportant un insert d'eau 41' et un insert 42' de forte concentration de $K_2HPO_4$. On mesure les densités de plusieurs inserts 39 de concentrations différentes et celles des inserts 41' et 42' en l'absence et en présence d'un sachet d'eau 43 simulant la corpulence. Les changements de densités mesurées sur les inserts 41' et 42' selon la présence ou l'absence du sachet d'eau 43 permettent de calculer des corrections de la densité mesurée de la vertèbre du patient selon la corpulence de ce dernier.

FIG. 4

EP 0 409 698 A1

EP 0 409 698 A1

## PROCEDE DE CORRECTION DE LA MESURE DE LA DENSITE OSSEUSE DANS UN SCANNER

L'invention concerne les scanners à rayons X et, plus particulièrement dans tels scanners, un procédé de mesure de la densité osseuse d'un patient qui permet de corriger la mesure de densité en fonction de la corpulence du patient.

Pour examiner un patient, on utilise de plus en plus des appareils à rayons X appelés "scanners" qui réalisent des images de coupes transversales du patient. Ces appareils sont basés sur le phénomène physique d'absorption des rayons X par le corps humain. Cette absorption est directement liée à la distance parcourue x des rayons X dans le corps selon la formule :

$$I = I_o e^{-bx}$$

formule dans laquelle :

$I_o$ est l'intensité du rayonnement entrant dans le corps humain,

$I$ est l'intensité du rayonnement sortant du corps humain,

$b$ est un coefficient d'atténuation qui dépend du corps traversé.

Dans une échelle de mesure logarithmique, l'atténuation $I/I_o$ est égale à $bx$, c'est-à-dire qu'elle est proportionnelle à la distance x.

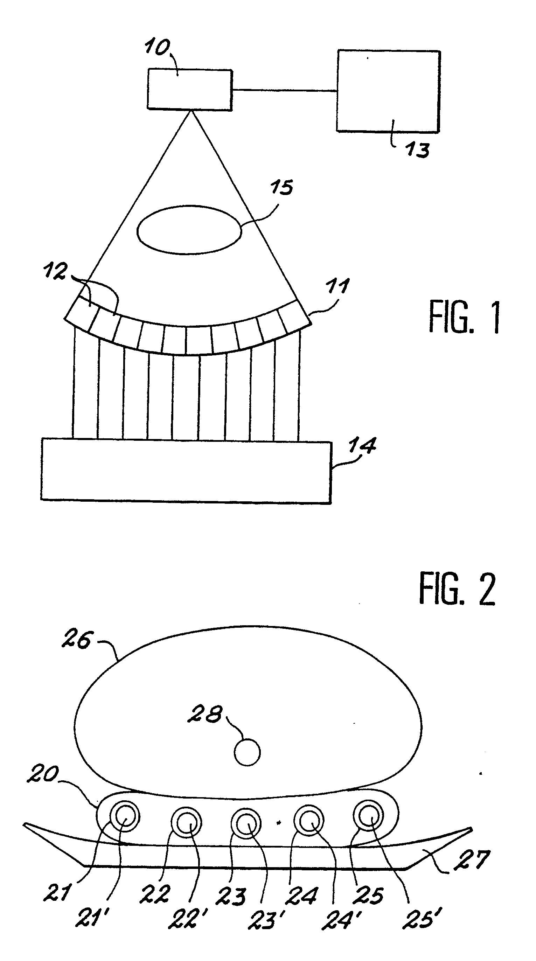

Ces appareils sont constitués essentiellement, comme le montre la figure 1, d'une source 10 de rayons X associée à un dispositif de détection 11, ces deux éléments étant disposés l'un par rapport à l'autre dans une relation géométrique fixe de manière à pouvoir intercaler entre eux un corps 15 à examiner. En outre, ils sont supportés par une structure (non représentée) qui peut tourner autour du corps à examiner de manière à irradier le corps suivant des angles différents. La source à rayons X, qui est commandée par un dispositif 13, émet ses rayons suivant un secteur angulaire qui a une largeur suffisante pour illuminer toute la section transversale du corps 15. Le dispositif de détection 11 a la forme d'un secteur annulaire dont la longueur est adaptée à la largeur du faisceau de rayons X et est constitué d'un grand nombre de détecteurs élémentaires 12 juxtaposés les uns à côté des autres.

Pour obtenir une image de la section transversale du corps 15 traversé par le faisceau de rayons X, on fait tourner la structure de support de la source 10 et du dispositif de détection 11 autour du corps 15 et on mesure les signaux de sortie des détecteurs élémentaires 12 pour les traiter de manière appropriée dans un dispositif électronique 14 selon des procédés connus afin d'en tirer une image représentative de la section transversale.

Chaque point élémentaire de l'image représentative de la section transversale a une luminosité dont la valeur indique l'absorption subie par le rayonnement X par cette partie correspondante de l'objet. Aussi, on a pensé à utiliser une telle image pour mesurer la densité osseuse du squelette d'un patient, en général en effectuant cette mesure pour les vertèbres lombaires.

Pour effectuer une mesure de densité osseuse, il est d'abord nécessaire d'effectuer un étalonnage de l'appareil à l'aide de plusieurs inserts d'une solution de di-potassium hydrogénophosphate ($K_2HPO_4$) dont le comportement énergétique est équivalent à celui de la partie spongieuse d'une vertèbre lombaire. En pratique, on utilise un fantôme 20 (figure 2) qui est placé sous un patient 26 dans la zone d'une vertèbre lombaire 28.

Ce fantôme 20 comporte, par exemple, cinq trous cylindriques 21 à 25 dans lesquels sont introduits des tubes cylindriques $21'$ à $25'$, l'un $21'$ contenant de l'eau et les quatre autres $22'$ à $25'$ contenant respectivement une solution de $K_2HPO_4$ à une concentration différente, par exemple 50 mg/cc, 100 mg/cc, 150 mg/cc et 200 mg/cc. Lorsque le patient 26 est en place sur un lit 27, on effectue un examen de manière à obtenir l'image d'une section transversale du corps du patient à l'endroit des vertèbres lombaires, cette image comportant la vertèbre lombaire 28 et les tubes ou inserts $21'$ à $25'$. L'image des inserts est de plus en plus claire selon que la concentration de la solution en $K_2HPO_4$ est plus élevée.

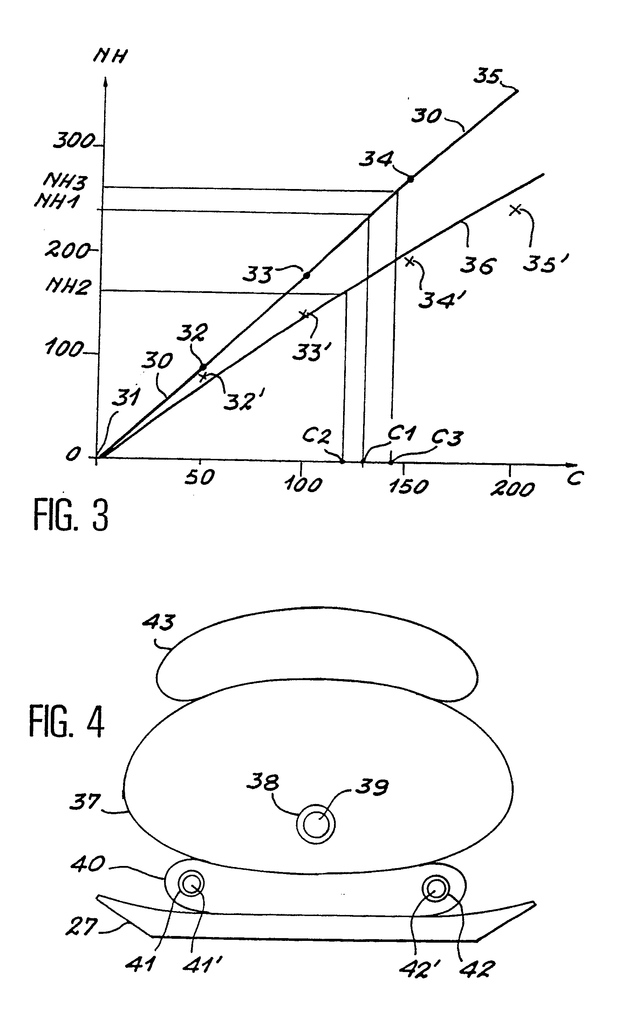

Comme chaque point élémentaire de l'image appelé pixel a une luminosité représentative d'une densité qui est mesurée suivant une échelle appropriée, en unités ou niveaux Hounsfield NH, il est possible de tracer la courbe 30 de la figure 3 qui relie les points 31 à 35 représentatifs des densités des inserts $21'$ à $25'$ mesurées en Niveaux Hounsfield NH. Ainsi, si pour la vertèbre 28, la mesure est NH1 sur l'axe des ordonnées, on en déduira que sa densité est équivalente à celle d'une concentration C1 de $K_2HPO_4$, ce qui correspond à une densité osseuse déterminée.

Le procédé de mesure de la densité osseuse d'une vertèbre qui vient d'être décrit succinctement manque de précision. Une des principales sources d'erreur est le fait que la corpulence du patient joue un rôle important sur la mesure de la densité des objets.

L'explication de ce phénomène est que le faisceau de rayons X utilisé par les scanners est polychromatique et avec un grand pourcentage de photons situés dans une zone spectrale de basse énergie. Le

2

procédé de reconstruction d'une image tient compte de ce fait et introduit une correction mathématique qui compense l'atténuation relativement plus forte des photons de basse énergie. Cette correction sera d'autant plus importante que l'atténuation est forte et il en est ainsi lorsque le patient est corpulent car le filtrage des photons de basse énergie par le corps humain est d'autant plus fort que ce dernier est volumineux.

Pour un scanner dont l'énergie de faisceau est homogène le long du dispositif de détection, la valeur corrigée $X'$ d'une atténuation $X$ sera donnée par la formule :

$$X' = X + A_1X^2 + A_2X^3 + ...A_nX^{n+1}$$

Les coefficients $A_1$, $A_2$...$A_n$ dépendent de nombreux paramètres tels que la nature de l'anode, de la nature de l'objet examiné, du dispositif de détection. On calcule ces coefficients pour le cas où le corps du patient examiné contient surtout des tissus constitués essentiellement d'eau. L'optimisation des coefficients est donc réalisée pour un fantôme d'eau afin que la densité mesurée sur l'image corrigée soit homogène sur toute sa surface.

Dans le cas où, dans le champ du faisceau de rayons X, se trouve un fantôme d'eau et un objet dont la masse atomique est différente de celle de l'eau, par exemple le calcium d'un os du squelette, les coefficients $A_1$, $A_2$...$A_n$ déterminés avec un fantôme d'eau ne sont plus valables pour un rayon du faisceau traversant en même temps le fantôme d'eau et l'objet.

En effet, le faisceau, reçu par le dispositif de détection et atténué par l'objet en calcium, correspondra à des chemins optiques dont les longueurs dans l'eau seront différentes. Or, plus le chemin dans l'eau sera long, plus l'énergie moyenne du faisceau sera décalée vers les hautes énergies, ce qui correspond à un durcissement du faisceau, de sorte que le faisceau sera moins atténué par l'objet en calcium. Il en résulte alors que, pour un patient corpulent, la mesure de la densité de calcium sera sous-estimée par rapport à celle d'un patient de moindre corpulence.

Il en résulte que la courbe d'étalonnage mesurée sera affectée par ce phénomène et donnera les points $32'$ à $35'$, correspondant à une courbe moyenne 36, qui donnent des densités plus faibles que les valeurs réelles.

Ainsi, le procédé actuel conduit à une erreur de mesure (C3 - C2) provenant, d'une part, de ce que la valeur mesurée NH2 correspond à une concentration C2 sur la courbe mesurée 36 et, d'autre part, de ce que cette valeur NH2 est sous-estimée et devrait être NH3 qui correspondrait à une concentration C3 sur la courbe théorique 30.

Le but de la présente invention est donc de mettre en oeuvre un procédé qui permet d'éliminer cette erreur de mesure du procédé traditionnel en tenant compte de la corpulence du patient.

L'invention se rapporte à un procédé de correction de la mesure de la densité osseuse dans un scanner à rayons X qui comprend les opérations suivantes :

a) la mise en place dans le champ du scanner d'un fantôme simulant le corps d'un patient de faible corpulence et comportant une cavité pour recevoir un insert simulant une vertèbre,

b) la mise en place sous le fantôme de simulation d'un fantôme d'étalonnage comportant deux cavités pour recevoir la première un insert contenant de l'eau et la deuxième un insert contenant une solution à forte concentration de di-potassium hydrogénophosphate ($K_2HPO_4$);

(c) la mise en place dans la cavité du fantôme de simulation d'un insert contenant une solution de $K_2HPO_4$ à une concentration AO et la mesure des densités suivantes :
- $V_c(1)$ de la solution contenue dans l'insert simulant la vertèbre,
- $D_1(E)$ de l'eau contenue dans le premier insert d'étalonnage, et
- $D_1(S)$ de la solution contenue dans le deuxième insert d'étalonnage;

(d) la mise en place d'un sachet d'eau sur le fantôme de simulation et la mesure des densités suivantes :
- $V_m(1)$ de la solution contenue dans l'insert simulant la vertèbre,
- $D'_1(E)$ de l'eau contenue dans l'insert d'étalonnage et,
- $D'_1(S)$ de la solution contenue dans l'insert d'étalonnage;

e) le calcul de $D_{HD}(1) = [D_1(S)-D_1(E)] - [D'_1(S)-D'_1(E)]$ qui indique le changement de la densité de la solution contenue dans le deuxième insert d'étalonnage dû à la présence du sachet d'eau par rapport à la densité du premier insert;

f) la réitération des opérations (c), (d) et (e) pour n inserts de simulation de la vertèbre contenant des solutions de concentrations A1 à An différentes de AO, de manière à obtenir pour chaque nouvelle concentration d'autres valeurs de $V_c$, $V_m$ et $D_{HD}$

g) le calcul des coefficients de correction $a_o$ à $a_n$ du polynôme de degré n tel que

$$V_C = V_m + \sum_{n=o}^{n} a_n V_C{}^{n} \cdot D_{HD}$$

Il comprend en outre, l'opération suivante :

(h) le calcul de la valeur moyenne des différences de densités $[D_{(i)}(S) - D_i(E)]$ entre la solution de $K_2HPO_4$ et l'eau pour les n inserts (39) simulant la vertèbre de manière à obtenir la différence :

$$D(S) - D(E) = \frac{\sum_{i=1}^{n} \left[ D_i(S) - D_i(E) \right]}{n}$$

Pour effectuer la correction lors de la mesure de la densité osseuse du patient, il faut compléter par les opérations suivantes :

(i) la mise en place du patient à la place du fantôme de simulation et la mesure des densités suivantes :
- $V_m(V)$ de la vertèbre du patient qui est en coïncidence avec le fantôme d'étalonnage,
- $D_m(E)$ de l'eau contenue dans le premier insert d'étalonnage;
- $D_m(S)$ de la solution contenue dans le deuxième insert,

(j) le calcul de la différence
$D_{HD} = [D(S) - D(E)] - [D_m(S) - D_m(E)]$

(k) le calcul de la valeur corrigée $V_c(Ver) > V_m(Ver)$ de la densité osseuse de la vertèbre (Ver) du patient qui satisfasse l'équation :

$$V_C(Ver) = V_m(Ver) + \sum_{n=o}^{n} a_n V_C{}^{n}(Ver) \cdot D_{HD}$$

les coefficients $a_n$ étant ceux obtenus par l'opération (g).

De préférence, la correction sera effectuée selon un polynôme de deuxième degré de sorte que l'étalonnage sera limité à la mise en place de trois inserts différents simulant la vertèbre.

D'autres caractéristiques et avantages de la présente invention apparaîtront à la lecture de la description suivante d'un exemple particulier de réalisation, ladite description étant faite en relation avec les dessins joints dans lesquels :
- la figure 1 est un schéma de principe d'un scanner à rayons X,
- la figure 2 montre les positions respectives du corps du patient et du fantôme avec des inserts d'étalonnage utilisés dans les procédés de l'art antérieur,
- la figure 3 montre, à l'aide de courbes, l'effet de la corpulence du patient sur la mesure de la densité osseuse, et
- la figure 4 montre le fantôme de simulation du corps du patient et celui d'étalonnage qui sont utilisés pour mettre en oeuvre le procédé selon l'invention.

Le procédé de correction selon l'invention est basé sur le fait que, pour un scanner donné et pour un fantôme d'étalonnage donné, l'écart de densité mesuré entre l'insert d'eau et celui de haute concentration devrait être une constante si le patient avait toujours la même corpulence. Quand cet écart change, ce ne peut être dû qu'à la corpulence du patient. Comme cette corpulence affecte également la valeur mesurée $V_m$ de la densité de la vertèbre, on exploite le changement de densité $D_{HD}$ mesurée sur l'insert de haute concentration dû à la corpulence afin de corriger la densité mesurée sur la vertèbre et obtenir la valeur corrigée $V_c$.

Si les valeurs $V_c$, $V_m$ et $D_{DH}$ sont exprimées en Niveaux Hounsfield NH, la correction proposée sera donc de la forme :
$V_c = V_m + K(V_c) \cdot D_{HD}$    (1)

Dans le cas où $K(V_c)$ est un polynôme du deuxième degré, on peut écrire.
$V_c = V_m + (a + bV_c + ev_c^2)D_{HD}$    (2)
ou
$eD_{HD} V_c^2 + (bD_{HD} - 1) V_c + aD_{HD} - V_m = 0$    (3)
La valeur $V_c$ qui sera la solution de l'équation 3 devra être positive et supérieure à $V_m$.

4

Pour déterminer les valeurs des coefficients a, b et e du polynôme $K(V_c)$, l'invention propose d'effectuer les opérations qui vont être décrites ci-après.

La première opération consiste à placer, dans le champ du scanner un fantôme 37 (figure 4) simulant le corps d'un patient de faible corpulence et comportant une cavité 38 pour recevoir un insert 39 simulant une vertèbre lombaire. Sous ce fantôme 37 de simulation du corps du patient, on place un fantôme d'étalonnage 40 présentant deux cavités 41 et 42 pouvant contenir l'une un insert 41' rempli d'eau et l'autre un insert 42' rempli d'une solution de di-potassium hydrogénophosphate ($K_2HPO_4$) à forte concentration, par exemple 250 mg/cc.

Pour une première série de mesures, on introduit dans le fantôme de simulation un insert 39 contenant une solution à faible concentration de $K_2HP_{04}$, par exemple 50 mg/cc. Sur l'image obtenue après un tour d'exploration du scanner, on mesure les densités suivantes :
- $V_c(1)$ de la solution contenue dans l'insert 39 simulant la vertèbre lombaire,
- $D_1(E)$ de l'eau contenue dans l'insert 41',
- $D_1(S)$ de la solution contenue dans l'insert 42'.

On place ensuite un sachet d'eau 43 sur le fantôme de simulation 37 pour simuler, en combinaison avec le fantôme 37, un patient de forte corpulence. Après un tour d'exploration du scanner, on mesure les densités suivantes :
- $V_m(1)$ de la solution contenue dans l'insert 39,
- $D'_1(E)$ de l'eau contenue dans l'insert 41',
- $D'_1(S)$ de la solution contenue dans l'insert 42'.

Le calcul $[D_1(S) - D_1(E)] - [D'_1(S) - D'_1(E)]$ donnera la première valeur $D_{HD}(1)$ de $D_{HD}$.

Cette première série de mesures est répétée pour un insert 39 de concentration moyenne, par exemple 125 mg/cc de manière à obtenir des valeurs $V_c(2)$, $V_m(2)$ et $D_{HD}(2)$, puis pour un insert de haute concentration, par exemple 200 mg/cc, de manière à obtenir des valeurs $V_c(3)$, $V_m(3)$ et $D_{HD}(3)$.

Les différentes valeurs de $V_m$, $V_c$ et $D_{HD}$ qui ont été obtenues par les opérations décrites ci-dessus permettent de calculer les coefficients a, b et e du polynôme $K(V_c)$ et donc de définir l'équation (2) pour effectuer les corrections quand on effectue des mesures de densités osseuses sur les patients.

Le procédé de calcul des coefficients a, b et e a été exposé dans le cas où $K(V_c)$ est un polynôme du deuxième degré. Cependant, $K(V_c)$ peut être un polynôme de degré n supérieur à deux et les différentes opérations du procédé de calcul des $(n+1)$ coefficients $a_0$ $a_n$ du polynôme sont alors les suivantes :

(a) La mise en place dans le champ du scanner d'un fantôme (37) simulant le corps d'un patient de faible corpulence et comportant une cavité (38) pour recevoir un insert (39) simulant une vertèbre;

(b) la mise en place sous le fantôme de simulation d'un fantôme d'étalonnage (40) comportant deux cavités (41,42) pour recevoir la première un insert (41') contenant de l'eau et la deuxième un insert (42') contenant une solution à forte concentration de di-potassium hydrogénophosphate ($K_2HPO_4$);

(c) la mise en place dans la cavité (38) d'un insert (39) contenant une solution de $K_2HPO_4$ à une concentration AO et la mesure des densités suivantes :
- $V_c(1)$ de la solution contenue dans l'insert (39) simulant la vertèbre,
- $D_1(E)$ de l'eau contenue dans le premier insert d'étalonnage (41'), et
- $D_1(S)$ de la solution contenue dans le deuxième insert d'étalonnage (42');

(d) la mise en place d'un sachet d'eau (43) sur le fantôme de simulation (37) et la mesure des densités suivantes :
- $V_m(1)$ de la solution contenue dans l'insert (39) simulant la vertèbre,
- $D'_1(E)$ de l'eau contenue dans l'insert d'étalonnage (41'), et
- $D'_1(S)$ de la solution contenue dans l'insert d'étalonnage (42');

(e) le calcul de
$D_{HD}(1) = [D_1(S) - D_1(E)] - [D'_1(S) - D'_1(E)]$
qui indique le changement de la densité de la solution contenue dans le deuxième insert d'étalonnage (42') dû à la présence du sachet d'eau (43).

(f) la réitération des opérations (c), (d) et (e) pour n inserts (39) de simulation de la vertèbre contenant des solutions de concentrations A1 à An différentes de AO, de manière à obtenir pour chaque nouvelle concentration d'autres valeurs de
$V_c$, $V_m$ et $D_{HD}$

(g) le calcul des coefficients de correction $a_0$ à $a_n$ du polynôme de degré n tel que

$$V_c = V_m + \sum_{n=o}^{n} a_n V_c^{n} \cdot D_{HD}$$

Au cours des mesures de densités osseuses sur les patients, il faut mettre en place le fantôme d'étalonnage sous le patient et mesurer, d'une part, la densité $V_m$ de la vertèbre et, d'autre part, le changement de densité $D_{HD}$ mesurée sur l'insert $42'$ de haute concentration par rapport à l'eau. Connaissant les coefficients a, b et e par le procédé décrit ci-dessus et $V_m$ et $D_{DH}$ résultant de la mesure sur le patient, l'équation (3) permet de calculer $V_c$.

Pour mesurer le changement de densité $D_{HD}$ mesuré sur l'inserte $42'$ de haute concentration par rapport à l'eau, il faut calculer la différence

$[D(S) - D(E)] - [D_m(S) - D_m(E)]$

c'est-a-dire la différence entre deux termes dont le premier $[D(S) - D(E)]$ est la différence de densités entre la solution de $K_2HPO_4$ et l'eau du fantôme d'étalonnage en l'absence de sachet d'eau dans le cas des mesures avec le fantôme de simulation du corps du patient tandis que le deuxième terme

$[D_m(S) - D_m(E)]$ est la différence de densités entre la solution de $K_2HPO_4$ et l'eau dans le cas où le corps du patient remplace le fantôme de simulation le fantôme d'étalonnage restant en place.

Il est à remarquer que les éléments du premier terme $[D(S) - D(E)]$ sont mesurés lors des opérations d'étalonnage pour chacun des n inserts (39) de simulation de la vertèbre en l'absence de sachet d'eau et les valeurs ainsi mesurées sont affectées par les concentrations différentes des solutions contenues dans l'insert (39) simulant la vertèbre. Cet effet est cependant très faible par rapport à celui de la corpulence du patient et l'invention propose de calculer par une opération répertoriée (h) la valeur moyenne de ce premier terme, c'est-à-dire la valeur moyenne des différences $D_1(S) - D_1(E)$, $D_2(S) - D_2(E)$,....$D_n(S) - D_n(E)$ que l'on exprime par :

$$\frac{\sum_{i=1}^{n} \left[ D_i(S) - D_i(E) \right]}{n} = D(S) - D(E)$$

Au lieu de calculer la valeur moyenne, on pourrait ne retenir qu'une des différences $D_i(S) - D_{(i)}(E)$ parmi n, par exemple celle qui est obtenue avec l'insert correspondant à la densité osseuse la plus usuelle. Lors de la mesure de la densité osseuse du patient, les opérations complémentaires à effectuer pour obtenir une valeur corrigée de la densité osseuse du patient sont alors les suivantes :

(i) La mise en place du patient à la place du fantôme de simulation et la mesure des densités suivantes :
- $V_m$(Ver) de la vertèbre du patient qui est en coïncidence avec le fantôme d'étalonnage (40),
- $D_m(E)$ de l'eau contenue dans le premier insert d'étalonnage $(41')$,
- $D_m(S)$ de la solution contenue dans le deuxième insert $(42')$,

(j) le calcul de la différence

$D_{HD} = [D(S) - D(E)] - [D_m(S) - D_m(E)]$

(k) le calcul de la valeur corrigée $V_c$(Ver) > $V_m$(Ver) de la densité osseuse de la vertèbre (Ver) du patient qui satisfasse l'équation :

$$V_c(Ver) = V_m(Ver) + \sum_{n=o}^{n} a_n V_c^{n}(Ver) \cdot D_{HD}$$

les coefficients $a_n$ étant ceux obtenus par l'opération (g).

Le procédé qui vient d'être décrit permet donc de corriger la valeur mesurée $V_m$ de la densité osseuse du patient pour obtenir la valeur corrigée $V_c$ en fonction de la corpulence du patient, corpulence qui est mesurée par $D_{HD}$.

Le procédé est mis en oeuvre, en l'absence du patient, à l'aide d'un fantôme de simulation du patient incluant des inserts simulant la vertèbre et d'un fantôme d'étalonnage ne comportant que deux inserts. Il faut en outre utiliser un sachet d'eau pour simuler la corpulence du patient. Il s'agit donc de mesures d'étalonnage qui sont effectuées pour une configuration donnée du scanner de manière à définir les coefficients a, b et e du polynôme de correction de degré 2.

Lors des mesures de la densité osseuse sur un patient, on n'a besoin que du fantôme d'étalonnage avec les deux inserts 41' et 42' et non pas les quatre ou cinq inserts du procédé traditionnel. En outre, il n'y a pas d'étalonnage à effectuer au cours des mesures sur le patient.

Le procédé a été décrit principalement en supposant une correction à l'aide d'un polynôme du deuxième degré mais on a montré que la correction peut être effectuée à l'aide d'un polynôme de degré quelconque n. Dans ce cas, comme cela a été indiqué ci-dessus, il faut utiliser $(n+1)$ inserts (39) de concentrations différentes A0, A1...An, de manière à obtenir $(n+1)$ équations qui permettent de déterminer les $(n+1)$ coefficients $a_o$ à $a_n$ du polynôme

$$\sum_{n=o}^{n} a_n \, V_C^{\phantom{C}n}$$

## Revendications

1. Procédé de correction de la mesure de la densité osseuse dans un scanner à rayons X qui comprend les opérations suivantes :

(a) la mise en place dans le champ du scanner d'un fantôme (37) simulant le corps d'un patient de faible corpulence et comportant une cavité (38) pour recevoir un insert (39) simulant une vertèbre;

(b) la mise en place sous le fantôme de simulation d'un fantôme d'étalonnage (40) comportant deux cavités (41,42) pour recevoir la première un insert (41') contenant de l'eau et la deuxième un insert (42') contenant une solution à forte concentration de di-potassium hydrogénophosphate ($K_2HPO_4$);

(c) la mise en place dans la cavité (38) d'un insert (39) contenant une solution de $K_2HPO_4$ à une concentration A0 et la mesure des densités suivantes :
- $V_c(1)$ de la solution contenue dans l'insert (39) simulant la vertèbre,
- $D_1(E)$ de l'eau contenue dans le premier insert d'étalonnage (41'), et
- $D_1(S)$ de la solution contenue dans le deuxième insert d'étalonnage (42');

(d) la mise en place d'un sachet d'eau (43) sur le fantôme de simulation (37) et la mesure des densités suivantes :
- $V_m(1)$ de la solution contenue dans l'insert (39) simulant la vertèbre,
- $D'_1(E)$ de l'eau contenue dans l'insert d'étalonnage (41'), et
- $D'_1(S)$ de la solution contenue dans l'insert d'étalonnage (42');

(e) le calcul de
$D_{HD}(1) = [D_1(S) - D_1(E)] - [D'_1(S) - D'_1(E)]$
qui indique le changement de la densité de la solution contenue dans le deuxième insert d'étalonnage (42') dû à la présence du sachet d'eau (43).

(f) la réitération des opérations (c), (d) et (e) pour n inserts (39) de simulation de la vertèbre contenant des solutions de concentrations A1 à An différentes de A0, de manière à obtenir pour chaque nouvelle concentration d'autres valeurs de
$V_c$, $V_m$ et $D_{HD}$

(g) le calcul des coefficients de correction $a_o$ à $a_n$ du polynôme de degré n tel que

$$V_C = V_m + \sum_{n=o}^{n} a_n \, V_C^{\phantom{C}n} \cdot D_{HD}$$

2. Procédé de correction selon la revendication 1, caractérisé en ce qu'il comprend, en outre, l'opération suivante :

(h) le calcul de la valeur moyenne des différences de densités $[D_{(i)}(S) - D_i(E)]$ entre la solution de $K_2HPO_4$ et l'eau pour les n inserts (39) simulant la vertèbre de manière à obtenir la différence :

EP 0 409 698 A1

$$\frac{\sum_{i=1}^{n} \left[ D_i(S) - D_i(E) \right]}{n} = D(S) - D(E)$$

3. Procédé de correction selon la revendication 2, caractérisé en ce qu'il comprend, lors de la mesure de la densité osseuse du patient, les opérations complémentaires suivantes :

(i) La mise en place du patient à la place du fantôme de simulation et la mesure des densités suivantes :
- $V_m(Ver)$ de la vertèbre du patient qui est en coïncidence avec le fantôme d'étalonnage (40),
- $D_m(E)$ de l'eau contenue dans le premier insert d'étalonnage (41'),
- $D_m(S)$ de la solution contenue dans le deuxième insert (42'),

(j) le calcul de la différence
$D_{HD} = [D(S) - D(E)] - [D_m(S) - D_m(E)]$

(k) le calcul de la valeur corrigée $V_c(Ver) > V_m(Ver)$ de la densité osseuse de la vertèbre (Ver) du patient qui satisfasse l'équation :

$$V_c(Ver) = V_m(Ver) + \sum_{n=o}^{n} a_n \, V_c^{\,n}(Ver) . D_{HD}$$

les coefficients $a_n$ étant ceux obtenus par l'opération (g).

4. Fantôme d'étalonnage (40) pour mettre en oeuvre le procédé de correction selon l'une quelconque des revendications précédentes, caractérisé en ce qu'il comprend une première cavité (41) destinée à recevoir un insert (41') rempli d'eau et une deuxième cavité (42) destinée à recevoir un insert (42') rempli d'une solution de di-potassium hydrogérophosphate.

8

FIG. 1

FIG. 2

FIG. 3

FIG. 4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-218367 (PICKER INTERNATIONAL, INC.)<br>* le document en entier *<br>--- | 1, 3 | A61B6/00 |
| A | US-A-4352020 (I. HORIBA ET AL.)<br>* abrégé *<br>* colonne 3, lignes 14 - 41 *<br>* colonne 5, ligne 21 - colonne 10, ligne 19 *<br>--- | 1, 3 | |
| A | US-A-4233507 (D.J. VOLZ)<br>* le document en entier *<br>----- | 1, 4 | |

DOMAINES TECHNIQUES RECHERCHES (Int. Cl.5)

A61B
G09B

Le présent rapport a été établi pour toutes les revendications

| Lieu de la recherche | Date d'achèvement de la recherche | Examinateur |
|---|---|---|
| LA HAYE | 17 SEPTEMBRE 1990 | FERRIGNO A. |

EPO FORM 1503 03.82 (P0402)